Europäisches Patentamt

European Patent Office

Office européen des brevets

(19)

(11) Publication number: **0 169 006**
A2

# EUROPEAN PATENT APPLICATION

(12)

(21) Application number: **85304711.6**

(22) Date of filing: **02.07.85**

(51) Int. Cl.⁴: **C 12 N 15/00,** C 12 N 1/00,
C 12 N 1/20, C 12 P 21/02,
C 07 K 13/00

(30) Priority: **13.07.84 GB 8417915**

(43) Date of publication of application: **22.01.86**
**Bulletin 86/4**

(84) Designated Contracting States: **AT BE CH DE FR GB IT
LI LU NL SE**

(71) Applicant: **IMPERIAL CHEMICAL INDUSTRIES PLC,
Imperial Chemical House Millbank, London SW1P 3JF
(GB)**

(72) Inventor: **Lord, John Michael, 48 Braemar Road,
Lillington Leamington Spa CV32 7EY (GB)**
Inventor: **Roberts, Lynne Margaret, 48 Braemar Road,
Lillington Leamington Spa CV32 7EY (GB)**
Inventor: **Atherton, Keith Thurston, 33 Clifton Road,
Runcorn Cheshire WA7 4SX (GB)**
Inventor: **Sharpe, Geoffrey Sydney, Cranmer The
Paddock, Helsby Cheshire WA6 9PQ (GB)**
Inventor: **Windass, John David, 3 Strathmore Close,
Holmes Chapel Cheshire CW4 7PP (GB)**
Inventor: **Tonge, David William, 98 Townfield Lane,
Barnton Northwich Cheshire CW8 4QL (GB)**

(74) Representative: **Thomas, Ieuan et al, Imperial Chemical
Industries PLC Legal Department: Patents PO Box 6,
Welwyn Garden City Herts, AL7 1HD (GB)**

(54) **Genetically modified micro-organisms.**

(57) A recombinant DNA molecule which (a) comprises (i) a gene which codes for proricin or a portion thereof and (ii) a suitable vector which carries the said gene adjacent to and under the control of appropriate expression signals; and (b) is capable, when inserted into a suitable host organism, of producing a metabolite which is or is closely related to proricin or a portion thereof.

Z/QM.33179 | EP

GENETICALLY MODIFIED MICROORGANISMS

This invention relates to the genetic modification of microorganisms, to novel microorganisms so obtained, to novel DNA sequences employed in the modification, and to metabolite produced by said novel microorganisms.

The techniques of recombinant genetic technology have been discussed in the literature, including the patent literature, and usually comprise, at their simplest, the steps of obtaining, from a donor cell, the DNA coding for a desired metabolite, cleaving a suitable vector to provide an insertion site wherein the aforesaid DNA may be inserted into the vector, inserting the aforesaid DNA into the vector to form a recombinant DNA molecule, genetically modifying a host bacterium, to which the aforesaid DNA is foreign, by introducing the recombinant DNA molecule into the host and culturing the genetically modified bacterium to cause it to produce the desired metabolite.

Ricin, and ricin-type molecules, e.g. abrin, modeccin and viscumin, are known compounds which are produced by living plant cells, and are known to be toxins. Toxins of this type consist of two polypeptide chains (known as the A and B chains) which are linked by a disulphide bridge and which, in ricin, are N-glycosylated. One of the chains, the A chain, is primarily responsible for the cytotoxic properties of the toxin. The other chain, the B chain, has sites, galactose binding sites which react with glycoproteins or glycolipids exposed at the cell surface, which enable the toxin to bind to cell surfaces; it also facilitates uptake of the molecule into the cell.

Ricin is produced in the plant Ricinus Communis, also known as the castor oil plant. It is believed that in the plant cell in which ricin is produced a so-called "preproricin" polypeptide is first produced. The preproricin comprises a leader sequence, the ricin A chain, a connecting sequence and the ricin B chain. It is believed that during transport within the plant cell the leader sequence is eliminated from the preproricin with formation of proricin, the A and B chains are joined by a disulphide bridge and the connecting sequence is eliminated to form mature ricin.

It has already been suggested that the toxicity of the ricin A chain might be exploited in anti-tumour therapy, by replacing the indiscriminately-binding B chain with a different carrier having the ability to bind only to tumour cells. Thus, various immunotoxins have already been prepared consisting of a conjugate of mature ricin or a ricin A chain and a tumour-specific monoclonal antibody. It has also been suggested that mature ricin may be converted into an immunotoxin under conditions which reduce or eliminate the problems associated with the non-specificity of the galactose binding sites.

In the second Applicant's published European patent application No 84304801.8 are disclosed recombinant DNA molecules which comprise a DNA sequence which codes for a polypeptide which is or is closely related to preproricin. In such recombinant DNA molecules, the aforesaid DNA coding sequence is inserted into a gene which is already in the vector, for example within the gene coding for ampicillin resistance in pBR322.

In the aforesaid European patent application it is proposed to modify the DNA sequence or part of

-3-

the DNA sequence coding for the B chain of ricin to eliminate or inactivate the galactose binding sites.

We have now devised methods for the preparation of recombinant DNA molecules, which are capable of being expressed in an appropriate host organism to give proricin, or portions thereof.

According to a first aspect of the present invention there is provided a recombinant DNA molecule which (a) comprises (i) a gene which codes for a polypeptide which is or is closely related to proricin, or a portion thereof and (ii) a suitable vector which carries the said gene adjacent to and under the control of appropriate expression signals and (b) is capable, when inserted into a suitable host organism, of producing a metabolite which is or is closely related to proricin, or a portion thereof.

By appropriate expression signals we mean powerful promoter sequences including a transcription initiation site and encoding an effective translation initiation region which comprises a ribosome binding site (Shine-Dalgarno sequence) and an appropriately placed translation initiation codon.

According to a second aspect of the present invention there is provided a genetically modified organism which comprises a recombinant DNA molecule according to the first aspect of the present invention.

According to a third aspect of the present invention there is provided a process for the production of a metabolite which comprises a polypeptide sequence which is or is closely related to proricin or a portion thereof which process comprises the step of culturing a genetically modified organism as defined in the second aspect of the present invention under conditions such that the said metabolite is produced.

The gene which codes for the said portion of proricin may code for the A chain or preferably the B chain or a truncated portion of the B chain of ricin from the N-terminal end of which about the first four amino acids are missing.

In the drawings accompanying this specification:

Figure 1 illustrates the nucleotide sequence of a preferred preproricin gene;

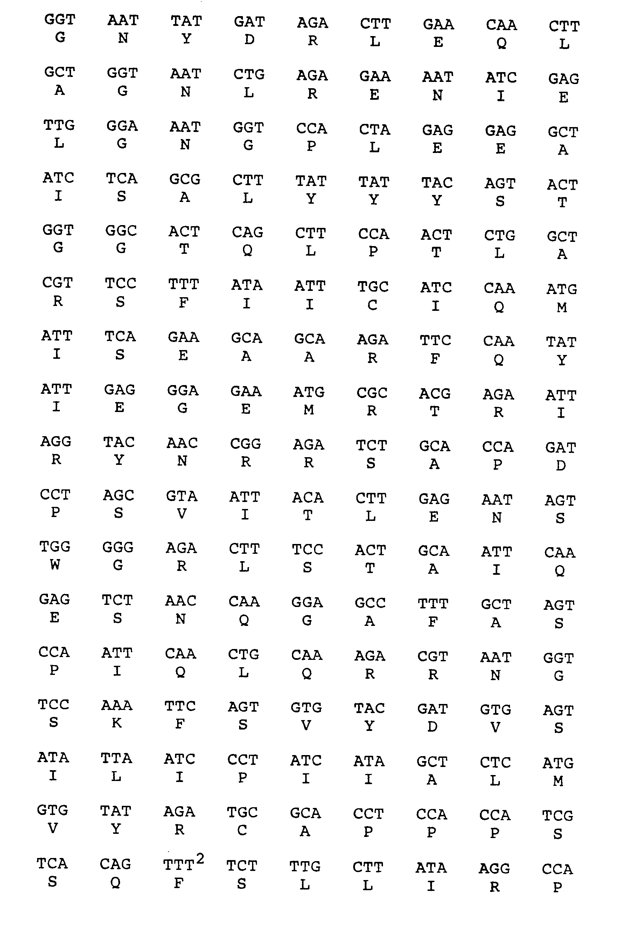

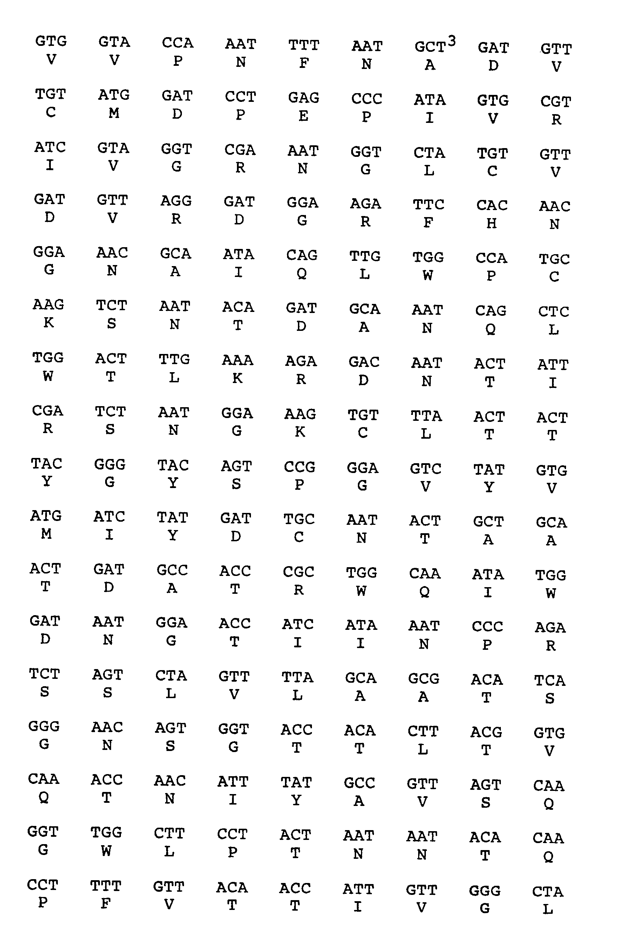

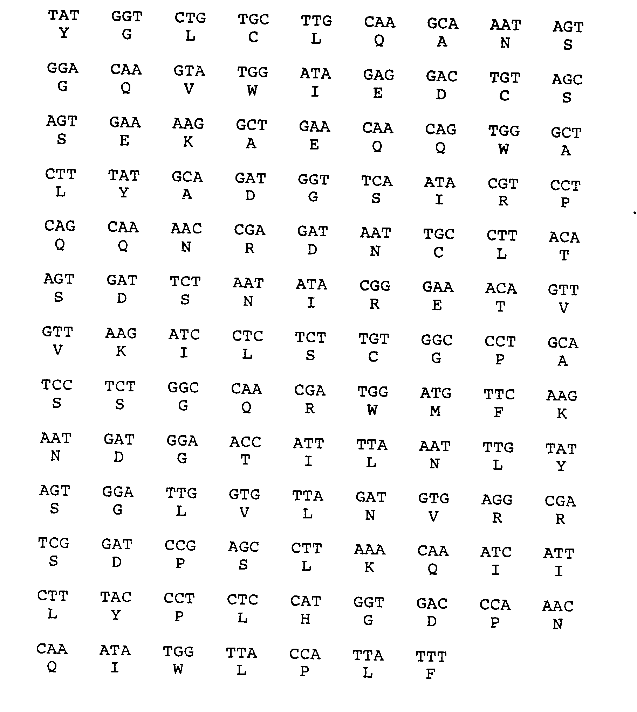

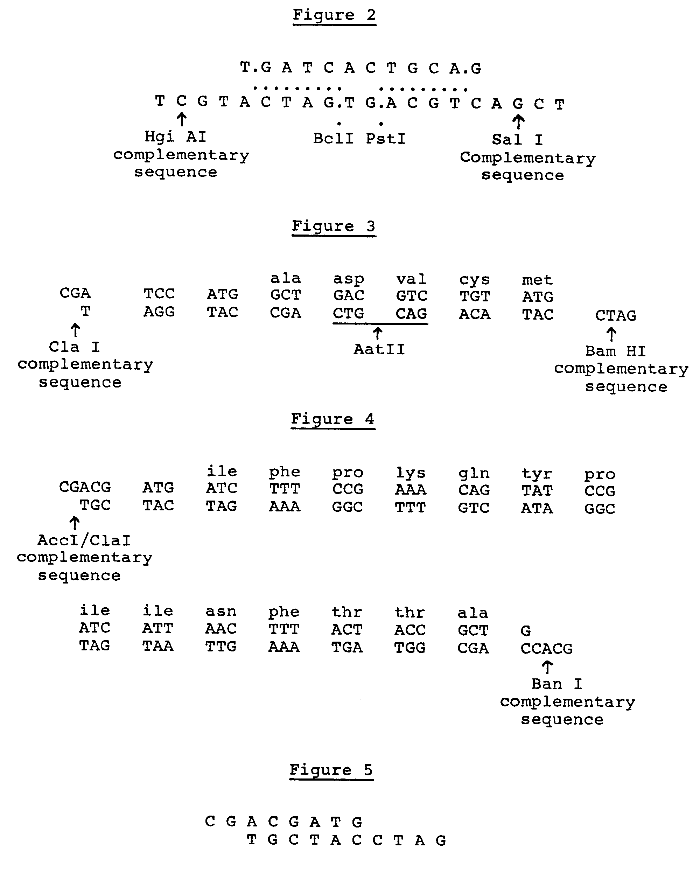

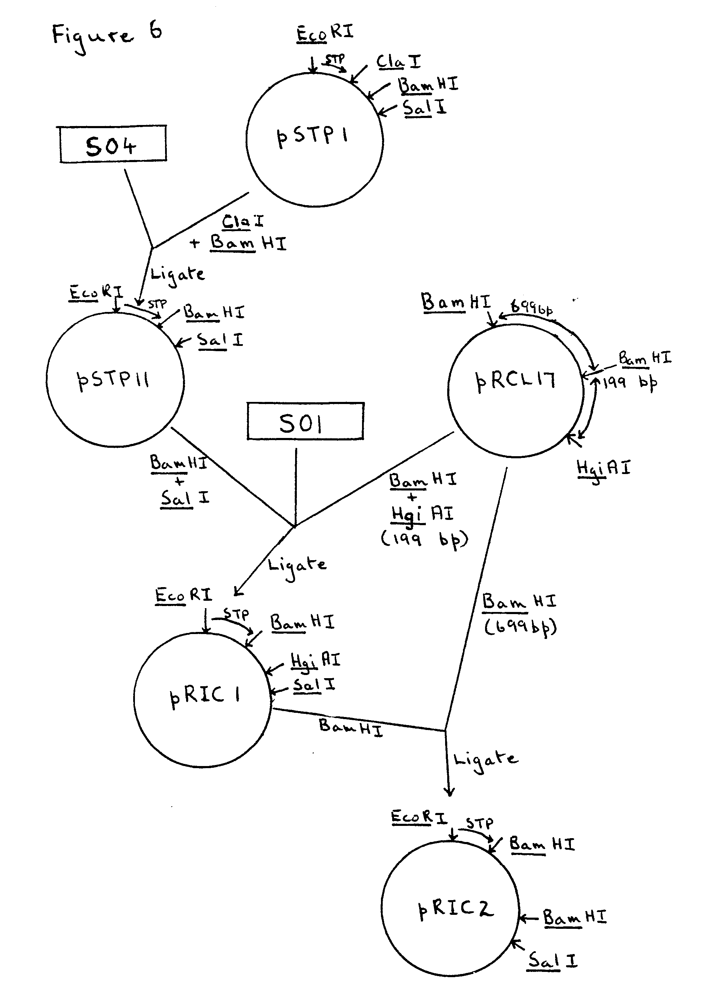

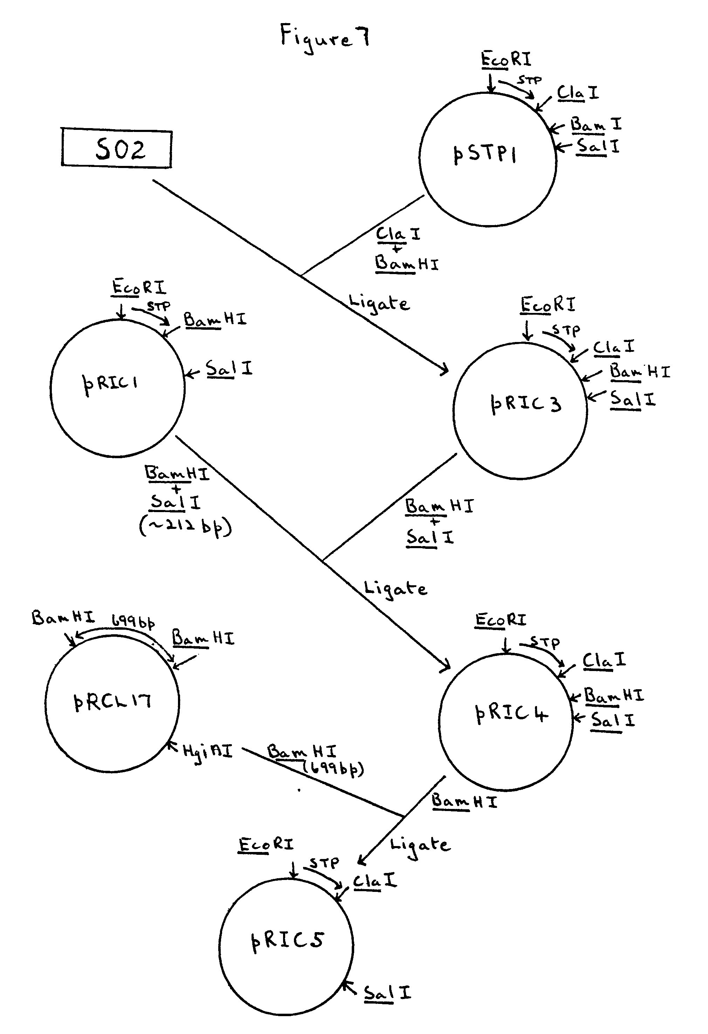

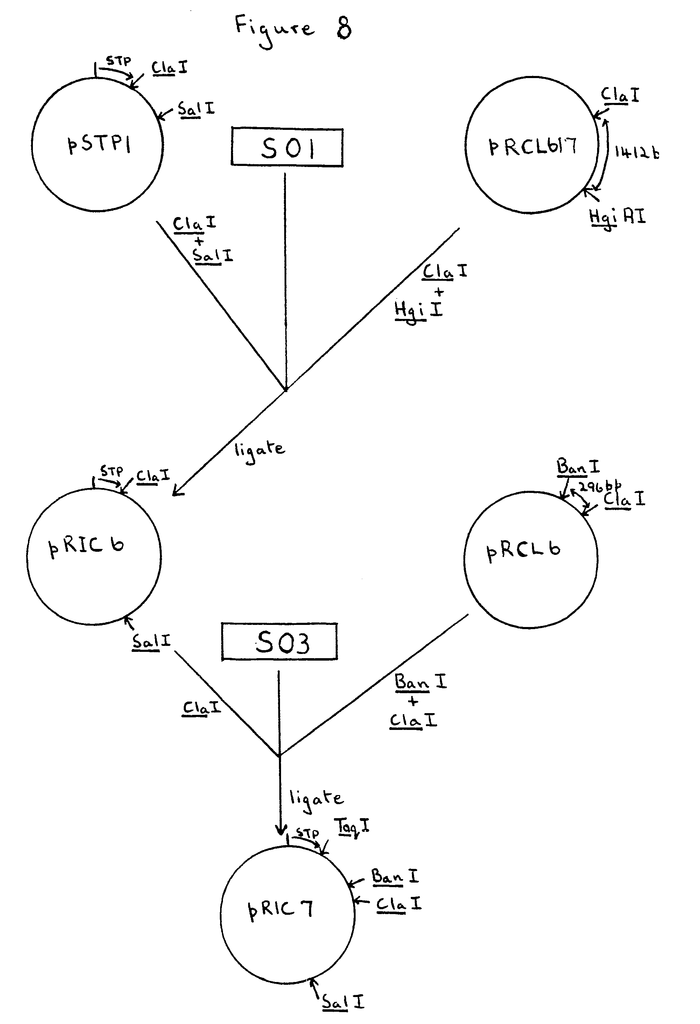

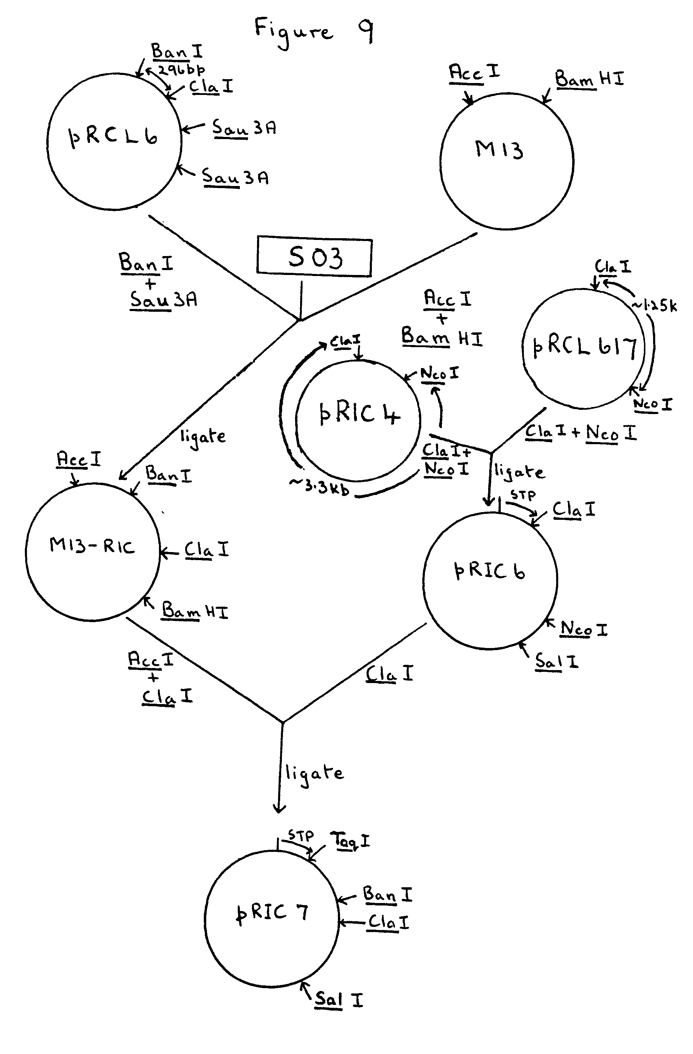

Figures 2, 3, 4 and 5 illustrate synthetic oligonucleotides, SO1, SO2, SO3 and SO4 respectively, which are useful for the preparation of recombinant DNA molecules according to the present invention;

Figures 6 and 7 are schematic representations of the preparation of recombinant DNA molecules capable of expressing a truncated B chain of ricin which lacks the first four amino acids and the B chain of ricin respectively;

Figures 8 and 9 are schematic representations of the preparation of recombinant DNA molecules capable of expressing proricin.

In Figures 6-9, "STP" represents "synthetic trp promoter."

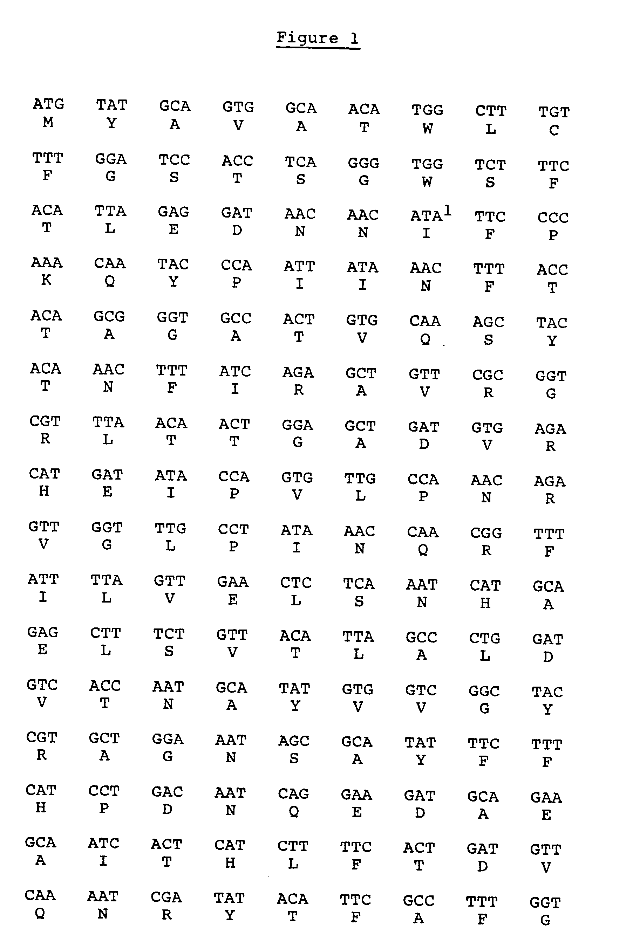

In Figure 1, the sequence is grouped into individual codons to assist interpretation and immediately under each codon is given the conventional one letter symbol (nomenclature of the IUPAC-IUB commission as published in the Biochemical Journal, 1969, Volume 113, pages 1-4) for the amino acid coded for by that codon. The first 24 codons code for a

-5-

leader sequence. The twenty-fifth codon, ATA, indicated by reference numeral 1 in the sequence, codes for the beginning of the A chain. The codon TTT, indicated by reference numeral 2 in the sequence, codes for the last amino acid (phenylalanine) of the A chain, and the codon GCT, indicated by reference numeral 3, codes for the first amino acid (alanine) of the B chain. The intervening twelve codons between reference numerals 2 and 3 code for the connecting sequence. It will be appreciated that by virtue of the degeneracy of the genetic code there are many nucleotide sequences which are equivalent to this sequence.

The genes of which recombinant DNA molecules according to the first aspect of the present invention are comprised are preferably obtained via recombination of appropriate fragments which are obtained by restriction enzyme digestion of the DNA sequences described in the aforesaid European patent application, the disclosure in which is incorporated herein by way of reference. However, we do not exclude the possibility that the previously described sequences might be modified by, for example, site directed mutagenesis. For example, the sequence AACATA, the ATA of which codes for the first amino acid of the A chain of ricin, might be amended to GATATC, i.e. creating an EcoRV restriction enzyme site, which can be cleaved with EcoRV to remove the leader sequence and leave the first codon for proricin, i.e. ATC (isoleucine), which can be cloned into a suitable vector adjacent to a translation initiation codon.

The vectors of which recombinant DNA molecules according to the first aspect of the present invention are comprised may be derived from suitable plasmids, e.g. pAT153, or suitable phages, e.g. M13.

Organisms from which the genetically modified organisms according to the second aspect of the present invention may be prepared may comprise plant cells, animal cells, or prokaryotic or eukaryotic cells. Preferably, however, the organism is a Gram negative bacterium, e.g. Alcaligenes eutrophus, Methylophilus methylotrophus or more preferably Escherichia coli.

The preparation of genetically modified organisms according to the second aspect of the present invention may be carried out by transformation, by which we mean in vitro introduction of "naked" DNA into the host organism, preferably after the host organism has been subjected to an appropriate treatment, e.g. treatment with a calcium chloride solution. Alternatively, the recombinant DNA molecule may be introduced into the host organism by transfer from another organism by cell conjugation, i.e. so-called conjugal transfer, which transfer may often be mediated by a conjugative plasmid.

In constructing coding sequences analogous to the coding sequences in the clones described in the aforesaid European patent application we have taken advantage of the degeneracy of the genetic code which permits substantial freedom in the choice of codons which can be used to construct the aforesaid genes. In particular, DNA sequences recognised by certain restriction enzymes were avoided if these enzymes were to be used at a later stage in the construction or were deliberately introduced where their presence might be beneficial at a later stage in the constructions. The codons were normally chosen as those preferred in the expression of genes in the appropriate microbial host, e.g. E. coli.

Genetically modified organisms according to the present invention may be employed in a variety of applications. Conveniently they will be used in a biosynthetic mode, in a suitable nutrient medium. The recombinant DNA molecule may be designed such that the metabolite is retained within the host cell, in which case the organism may be cultured and harvested as the intact cell with subsequent extraction of metabolite from the cells, for example after the cells have been separated from the medium. Alternatively, the metabolite may be extruded from the cell into the surrounding culture medium and extracted therefrom in the conventional way. Such techniques are well known.

The present invention will now be described by way of example.

Starting Materials

Recombinant DNA molecules

In the aforesaid European patent application, there is described the preparation and detection of clones which comprise recombinant DNA molecules formed by insertion of DNA sequences (obtained via reverse transcription of mRNA isolated from ripening Ricinus beans) coding for at least a portion of the preproricin gene into vectors pBR322 and pUC8. The determination of the inserted sequences in two of the clones is also described therein. The aforesaid two clones which contain recombinant DNA molecules pBRCL6 (which contains a 1614 base pair insert) and pBRCL17 (which contains a 1049 base pair insert) respectively in plasmid vector pBR322 were treated with PstI and the excised inserts were ligated with pUC8 which had been linearised with PstI, recombinant DNA molecules pRCL6 and pRCL17 respectively were obtained.

The insert in pRCL6 extends from nucleotide residue -102 to nucleotide residue 1512 and the insert in pRCL17 extends from nucleotide residue 733 to nucleotide residue 1782. In this numbering system, the nucleotide residues are numbered in the 5' to 3' direction with the first nucleotide residue of the codon specifying the amino terminal residue of mature ricin A chain numbered 1 and the nucleotide residues on the 5' side of nucleotide residue 1 indicated by negative numbers; the final nucleotide residue of the codon for the C-terminus amino acid of the B chain is 1623.

In the aforesaid European patent application there is further described the preparation of recombinant DNA molecule pRCL617 which contains the complete nucleotide sequence encoding preproricin. This was achieved by isolating a fragment, 323 base pairs in length, obtained from pRCL17 by digestion with PstI and Sau96I and ligating this fragment to a fragment 1561 base pairs in length obtained from pRCL6 by digestion with PstI followed by partial digestion with Sau96I as is more fully described in the aforesaid European patent application.

Synthetic oligonucleotides

Synthetic olignucleotides SO1,SO2,SO3 and SO4 were prepared by the procedures described by Gait et al, Nucleic Acids Research 1980, Volume 8, pages 1081-1096 and Markham et al, Nucleic Acids Research 1980, Volume 8, pages 5193-5205.

Aliquots (0.25 nmole) of each of the discrete strands of SO1,SO2 and SO4 were separately phosphorylated with 1 unit of T4-induced polynucleotide kinase (ex Bethesda Research Laboratories) in a solution (10 microlitres) containing 100 mM Tris.HCl (pH 7.5), 20mM MCl$_2$, 20mM dithiothreitol and 100mM ATP for 60 minutes at 37$^O$C, and 10 minutes at 65$^O$C followed by freezing at -20$^O$C for storage.

Aliquots (50 picomoles) of solutions of both phosphorylated strands for a particular synthetic oligonucleotide were diluted (1;5 by volume) with water; the diluted solutions were mixed, placed in a water bath at 100$^O$ C and allowed to cool to room temperature to anneal the strands .

SO3 was prepared as a double stranded molecule which was then phosphorylated under the conditions described above.

Example 1

This example illustrates the preparation of a recombinant DNA molecule which codes for a truncated B chain of ricin which lacks the first four amino acids from the N-terminal end of the B chain of ricin. The preparation is illustrated schematically in Figure 6.

Construction of an Expression Vector.

The vector plasmid pSTP1 (European Patent Specification No 0,062 971 A) is designed to accept DNA fragments cloned at the ClaI site adjacent to a synthetic trp promoter. To enable BamHI - generated DNA fragments to be cloned and expressed from the synthetic trp promoter, the ClaI - BamHI region of pSTP1 was replaced by synthetic oligonucleotide SO4.

Plasmid pSTP1 DNA (5 micrograms) was digested for 90 mins at 37$^O$C with restriction endonucleases ClaI and BamHI in a buffer solution (50mM Tris.HCl(pH 8.0), 10mM MgCl$_2$ and 50mM NaCl in water (hereinafter referred to for convenience as "Buffer Solution A")). The reaction was terminated by incubation at 65°C for 10 min then the DNA was subjected to electrophoresis through 1% low melting-point agarose (ex Sigma Chemical Company, catalogue No A-4018). After staining with ethidium bromide, the gel was exposed to U.V. light (330 nm) to visualize the bands. The larger band was excised, placed in an eppendorf tube with water (1 ml/g gel slice) and heated to 65°C for 5 min to melt the agarose and was then vortexed.

Ligation of the ClaI + BamHI - cut pSTP1 (approximately 80 ng) with a portion (5pmole) of phophorylated synthetic oligonucleotide S04 was achieved using 1 unit of T4 DNA ligase (ex Pharmacia Fine Chemicals AB) under the conditions recommended by the manufacturer.

The ligation mixture was used to transform E. coli strain JA221 [Clarke and Carbon: J. Mol. Biol 120 (1978) 517-532], to ampicillin(Ap)resistance (100 micrograms/ml) by the calcium chloride method described in "Molecular Cloning - A Laboratory Manual", by Maniatis et al, published by Cold Spring Harbor Laboratory, 1982 (hereinafter referred to for convenience as "Maniatis"), at pages 250-251.

50 Ap$^r$ colonies were picked off and the plasmid DNA, isolated from ten of these colonies by the rapid boiling method of Holmes and Quigley (Analytical Biochemistry, 1981, Volume 114, pages 193-197) was, in each case, found to be smaller than pSTP1 and could not be cut by ClaI. The plasmid isolated from one of the latter colonies was designated pSTP11, and the presence of S04 between the ClaI and BamHI sites of pSTP1 was confirmed by DNA sequence analysis. (Sanger et al, Proceedings of the National Academy of Science, USA, 1977 Volume 74, pages 5463-5467).

-11-

## Construction of pRIC1

pRIC1 is a plasmid which contains the C-terminus of the ricin B-chain coding sequence.

Plasmid pRCL17 DNA (10 micrograms) was digested for 90 min at 37°C with the restriction endonuclease HgiAI (New England Bio Labs) in 150mM NaCl, 10mM Tris. HCl (pH8.0), 10mM MgCl$_2$, 10mM DTT and 100 microgram/ml bovine serum albumin. After electrophoresis through 1% agarose the slowest moving band corresponding to the largest fragment was excised, the DNA was electroeluted, phenol extracted and precipitated with isopropanol. This HgiAI DNA fragment was re-dissolved in Buffer Solution A and was digested with 5 units of BamHI for 90 mins at 37°C and at 65°C for 10 min. The BamHI - digested DNA was subjected to electrophoresis through a 5% polyacrylamide gel where it produced a pattern of 3 bands. The smallest and largest bands corresponding to DNA fragment sizes of 199 and 699base pairs respectively were excised from the gel and the DNA was electroeluted, phenol extracted and precipitated with isopropanol. The DNA pellets were dried then re-dissolved in 10 microlitres of water just prior to use.

pSTP11 was digested with BamHI and SalI for 90 min at 37°C in Buffer Solution A. Separation of the larger BamHI-SalI fragment was achieved by electrophoresis through 1% low melting point agarose. The band was excised and treated as described above.

A portion (5 microlitres equivalent to 5 pmole) of phosphorylated synthetic oligonucleotide SO1, the larger BamHI-SalI fragment (2 microlitres equivalent to 80 ng) from pSTP11 and the 199bp BamHI-HgiAI fragment from pRCL17 (1 microlitre) were mixed in the presence of T4 DNA ligase and incubated overnight at room temperature as described above. The ligation mix was used to transform E.coli strain ED8654 (met, hsd R⁻, supE, SupF) to ampicillin resistance.

Four Apʳ colonies were obtained and the plasmid DNA isolated from each was analysed using restriction endonucleases BamHI, BstEII, NcoI and Pst I sites. Since the pRCL17 fragment contained an NcoI site and two BstEII sites and the synthetic oligonucleotide SO1 contained a PstI site, the analyses indicated that the required ligation had occurred. This was confirmed by DNA sequence analysis through the BamHI and SalI regions. One of the plasmids with the correct structure was designated pRIC1.

Construction of pRIC2

pRIC2 is a plasmid which contains a portion of the ricin B-chain coding sequence which lacks the first four amino acids from the amino end thereof.

Plasmid pRIC1 DNA (1 microgram) was linearized by digestion with BamHI for 90 min at 37°C in Buffer Solution A. After enzyme inactivation at 65°C for 10 minutes a sample (80 ng) of the BamHI - cut plasmid was ligated with a portion (2 microlitres) of the 699-bp BamHI - fragment from pRCL17 described above, at room temperature, overnight, in the presence of T4 DNA ligase. The ligation mixture was used to transform E.coli strain ED8654 to ampicillin resistance.

Several hundred colonies were picked off and their plasmid content screened by the aforementioned rapid boiling method. Five isolates, having plasmids larger than the parental pRIC1, were chosen and were analysed further using BamHI and EcoRI+BglII. Digestion with BamHI followed by agarose gel

-13-

electrophoresis revealed the presence of a 699-bp fragment. The use of digests with both EcoRI and BglII served to indicate the orientation of insertion of the BamHI fragment. One plasmid which had the fragment in the correct orientation to allow the correct reading - frame continuity through the carboxy - terminal fragment, was designated pRIC2.

E.coli minicell preparations are used to check the authenticity of expression from the cloned sequences of pRIC2.

Example 2

This example illustrates the preparation of a recombinant DNA molecule which codes for the B chain of ricin. The preparation is illustrated schematically in Figure 7.

Construction of pRIC3

pRIC3 is a plasmid containing a synthetic oligonucleotide which bears a translation initiation coden (ATG) and encodes the first five amino acids of the ricin B chain immediately downstream from a synthetic trp promoter sequence.

Plasmid pSTP1 was digested to completion using the restriction enzymes ClaI and BamH1 in conjunction. Digests were performed in Buffer Solution A at 37°C in accordance with the manufacturer's instructions for the restriction enzymes. After digestion, enzyme activity was destroyed by incubation at 65°C for 10 minutes. The effectiveness of the digestion was determined by agarose gel electrophoresis of a sample of the cut material.

Totally digested pSTP1 was ligated with the phosphorylated synthetic oligonucleotide SO2 using T4 DNA ligase in accordance with the manufacturer's

-14-

instructions.  Ligation reactions were incubated at 15°C overnight.  The ratio of plasmid to synthetic oligonucleotide was set at a level to favour the insertion of the synthetic oligonucleotide rather than reinsertion of the native ClaI - BamHI fragment from pSTP1.

Ligated material was then used to transform E.coli strain C600(thr, leu, thi, lac, supE, ton A) to ampicillin (Ap) resistance using the known calcium chloride method for preparing competent cells. Resulting transformants were subsequently screened for tetracycline (Tc) sensitivity (10 microgram/ml) (pSTP1 normally confers Tc resistance which is lost by replacement of the ClaI - Bam HI region).  Tetracycline sensitive transformants were then subjected to the aforementioned Holmes and Quigley rapid boiling method for plasmid DNA preparation and the resulting plasmid DNA was restricted with the enzyme AatII in accordance with the manufacturer's instructions.  Clones containing the synthetic oligonucleotide should possess two AatII sites as opposed to the single site present in pSTP1.  Of 9 Tc$^S$ clones screened, all appeared identical with respect to AatII digestion.

One of the latter clones was designated pRIC3 and its DNA sequence was verified by subcloning of the EcoRI to SalI region into the phage M13 and sequencing by the aforementioned chain termination method of Sanger. These manipulations were performed using the reagent kits provided by Amersham International in accordance with the protocols supplied.

Construction of pRIC4

pRIC4 is a plasmid which contains both the amino-terminal and carboxy-terminal regions of the ricin B chain.  Its construction involves replacement of the BamHI-SalI region of pRIC3 with the corresponding region from pRIC1.

Plasmid pRIC1, prepared as in Example 1, and plasmid pRIC3 were digested separately to completion using the enzyme BamHI in accordance with the manufacturer's instruction.  Enzyme activity was removed by phenol extraction with subsequent chloroform extraction of the aqueous phase to eliminate contaminating phenol (Maniatis page 458).  Digested plasmid DNA was then recovered by ethanol precipitation (Maniatis p461) and the re-extracted DNA pellets resuspended in double distilled water.  Each BamHI digested plasmid was then digested to completion with the enzyme SalI in accordance with the manufacturer's instructions.  Equal proportions of each plasmid were ligated together as previously described and ligated DNA used to transform E.coli C600 to Ap resistance.

Transformants were screened at random (no insertional inactivation of an antibiotic resistance marker can be used as both parent and recombinant plasmids are Tc$^S$) by the technique of Holmes and Quigley for the preparation of plasmid DNA followed by restriction analysis using the enzymes PstI and AatII. pRIC1 has two PstI sites but only one AatII site whereas pRIC3 has two AatII sites but a unique PstI site.  Plasmids with two sites for both PstI and AatII were regarded as putative pRIC 4 clones.  Of 15 transformants screened, 11 gave restriction patterns corresponding to those predicted for pRIC4 with the above enzymes.  Four of these isolates were taken for more complete restriction analysis using the enzymes AluI, HinfI and HphI on plasmid DNA prepared by the the alkaline lysis method of Birnboim and Doly according to the modification of Ish-Horowicz (Maniatis p368). Two of the 4 isolates gave restriction patterns corresponding to those predicted, and one of those two isolates was designated pRIC4.

Construction of pRIC5

pRIC5 is a recombinant DNA molecule which encodes the ricin B chain adjacent to a synthetic trp promoter. It is constucted by insertion of the 699bp BamHI fragment from pRCL17 into the BamHI site of pRIC4. Two strategies were employed.

Strategy I

Plasmid pRIC4 was digested with BamHI to completion in accordance with the manufacturer's instructions. Enzyme activity was destroyed by incubation at 65°C for 10 min. An aliquot of this digestion, containing 20 ng of plasmid DNA, was ligated with 100 ng of the 699bp fragment from pRCL17 prepared in Example 1. The ligation was catalysed by the enzyme T4 DNA ligase used in accordance with the manufacturer's instructions for 4 hours at 15°C.

Strategy II

Plasmid pRCL17 was digested with the enzymes BamHI and HaeII in conjunction in Buffer Solution A in accordance with the manufacturer's instructions. This double digestion leaves both the 699 bp and 199 bp BamHI fragments intact but further digests the remainder of pRCL17 at four positions. Thus, on ligation, the regeneration of pRCL17 or its parent plasmid pUC8 is minimised. BamHI/HaeII digested pRCL17 (100 ng) was ligated with BamHI digested pRIC4 (20 ng) at 15°C overnight.

DNA from both strategies was used separately to transform E.coli C600 to Ap resistance. Transformant colonies were screened by the filter hybridisation method of Grunstein and Hogness (proceedings of the National Academy of Science, USA, 1975, Volume 72, page 3961). The hybridisation probe was prepared by nick translation of the 699 bp BamHI fragment from pRCL17. Nick translation was performed using a reagent kit supplied by Amersham International incorporating $\gamma^{32}P$ dCTP as the radio labelled nucleotide. Separation of radio labelled DNA from unincorporated nucleotide was by fractionation through a column of Sephadex G50 (Pharmacia) followed by determination of the Cherenkov radiation in each fraction (250 microlitre) using an LKB Rackbeta liquid scintillation counter.

Twelve positively hydrising transformants were selected for further analysis by BamHI digestion of plasmid DNA prepared by the Holmes and Quigley method. Eight of these transformants were seen to possess a BamHI fragment which comigrated with the 699 bp BamHI fragment from pRCL17 on an agarose gel. The correct orientation of insertion of the BamHI fragment was determined by digestion with the enzymes BglII, KpnI and PvuI. One clone possessing the correct orientation of insertion to produce the complete coding sequence of the ricin B chain was designated pRIC5.

E.coli minicell preparations are used to check the authenticity of expression from the cloned sequences of pRIC5.

Example 3

Preparation of a recombinant DNA molecule coding for proricin

Construction of a recombinant DNA molecule coding for proricin is illustrated in Figure 8.

pSTP1 (European Patent Publication No. 0062971) is treated with restriction enzymes ClaI and SalI and the larger fragment isolated by known techniques, e.g. agarose gel electrophoresis. pRCL617 is digested with restriction enzymes ClaI and HgiAI and a 1412 base pair fragment is isolated by known techniques. A mixture of the aforesaid isolated fragments and phosphorylated synthetic oligonucleotide SO1, is ligated using T4 DNA ligase under known conditions. The ligated mixture is transformed into E.coli and colonies containing recombinant DNA molecules are screened for the desired sequence; i.e. pRIC6. (An alternative construction of pRIC6 is illustrated in Figure 9).

A 296 base pair fragment, obtained by digesting pRCL6 with restriction enzymes BanI and ClaI, pRIC6 linearised with ClaI and a synthetic oligonucleotide SO3, which codes for the first fourteen amino acids of ricin together with an additional translation initiation codon, are ligated as hereinbefore described and after appropriate screening a recombinant DNA molecule pRIC7 containing a sequence coding for proricin joined via a ATG translation initiation codon directly to a synthetic trp promotor is obtained.

Example 4

Preparation of a recombinant DNA molecule coding for proricin

An alternative construction of a recombinant DNA molecule coding for proricin is illustrated in Figure 9.

pRCL6 is digested with restriction enzymes BanI and Sau3A and a 548 base pair fragment is isolated.

The replicative form (RF) of a single stranded M13 phage vector, e.g. M13mp8, is digested with restriction enzymes AccI and BamHI. The 548 base pair fragment, the digested M13 and phophorylated synthetic oligonucleotide SO3 are mixed, ligated and pM13-RIC is isolated. This phage contains a regenerated DNA fragment encoding the first 197 amino acids of proricin preceded by a translation initiation codon and a conveniently positioned AccI site.

pRIC4, prepared as described in Example 2, is digested with ClaI and NcoI and the fragment of approximately 3.3 kilobases is isolated by known techniques. pRCL617 is digested with ClaI and NcoI and the fragment of approximately 1.25 kilobases is isolated as described above. The two aforesaid fragments are ligated and pRIC6 is obtained.

pRIC6, linearised with ClaI, and the smaller sequence obtained on digesting M13-RIC with restriction enzymes AccI and ClaI are ligated and after appropriate screening pRIC7 is obtained.

Use of M13 vectors containing a segment of the lac operon into which DNA fragments can be introduced, e.g. M13mp8, affords the advantages that (a), when used with suitable plates and host bacteria, e.g. JM101, recombinant DNA molecules are produced which are readily identifiable by the production of white plaques, while plaques of the vector are blue, thus facilitating recognition of potentially rare tripartite recombinant DNA molecules of the desired type and (b) the inserted region can be readily sequenced to confirm that the desired molecule has been obtained.

CLAIMS

1. A recombinant DNA molecule which
(a) comprises (i) a gene which codes for a polypeptide which is or is closely related to proricin or a portion thereof, and
(ii) a suitable vector which carries the said gene adjacent to and under the control of appropriate expression signals; and
(b) is capable, when inserted into a suitable host organism, of producing a metabolite which is or is closely related to proricin or a portion thereof.

2. A recombinant DNA molecule as claimed in Claim 1 wherein the gene has the formula shown in Figure 1 or at least a portion thereof or a gene which is equivalent thereto by virtue of the degeneracy of the genetic code.

3. A recombinant DNA molecule as claimed in Claim 1 wherein the said gene codes for the B chain of ricin .

4. A recombinant DNA molecule as claimed in Claim 1 wherein the said gene codes for a polypeptide which is a truncated B chain of ricin which lacks the first four amino acids at the N-terminal end of the B chain of ricin.

5. A recombinant DNA molecule as claimed in Claim 1 wherein the said suitable vector is derived from a plasmid or phage.

6. A recombinant DNA molecule as claimed in Claim 5 wherein the plasmid is pSTP1.

7. A recombinant DNA molecule as claimed in Claim 1 wherein the appropriate expression control signals comprise a synthetic trp promoter.

-21-

8.     A genetically modified organism which has been genetically modified by the incorporation therein of a recombinant DNA molecule as claimed in Claim 1.

9.     A genetically modified organism as claimed in Claim 8 which is derived from a gram negative bacterium.

10.     A genetically modified organism as claimed in Claim 9 wherein the bacterium is a strain of E.coli.

11.     A process for the preparation of a recombinant DNA molecule as claimed in Claim 1 which process comprises the steps of digesting pRCL17 with suitable restriction enzymes and inserting a desired fragment therefrom into a suitable vector.

12.     A process as claimed in Claim 11 wherein pRCL6 is digested with suitable restriction enzymes and inserting a desired fragment therefrom and the said desired fragment from pRCL17 into a suitable vector.

13.     A process for the preparation of a genetically modified microorganism as claimed in Claim 8 which process comprises infecting a suitable organism with a recombinant DNA molecule as claimed in Claim 1.

14.     A process as claimed in Claim 13 wherein the organism is E.coli.

15.     A process for the production of a metabolite which comprises a polypeptide sequence which is or is closely related to proricin or a portion thereof which process comprises the steps of culturing a genetically modified organism as claimed in Claim 8 under conditions such that the said metabolite is produced.

16.     Biologically pure proricin, B-chain of ricin or truncated B-chain of ricin.

Figure 1

| | | | | | | | | |
|---|---|---|---|---|---|---|---|---|
| ATG<br>M | TAT<br>Y | GCA<br>A | GTG<br>V | GCA<br>A | ACA<br>T | TGG<br>W | CTT<br>L | TGT<br>C |
| TTT<br>F | GGA<br>G | TCC<br>S | ACC<br>T | TCA<br>S | GGG<br>G | TGG<br>W | TCT<br>S | TTC<br>F |
| ACA<br>T | TTA<br>L | GAG<br>E | GAT<br>D | AAC<br>N | AAC<br>N | ATA[1]<br>I | TTC<br>F | CCC<br>P |
| AAA<br>K | CAA<br>Q | TAC<br>Y | CCA<br>P | ATT<br>I | ATA<br>I | AAC<br>N | TTT<br>F | ACC<br>T |
| ACA<br>T | GCG<br>A | GGT<br>G | GCC<br>A | ACT<br>T | GTG<br>V | CAA<br>Q | AGC<br>S | TAC<br>Y |
| ACA<br>T | AAC<br>N | TTT<br>F | ATC<br>I | AGA<br>R | GCT<br>A | GTT<br>V | CGC<br>R | GGT<br>G |
| CGT<br>R | TTA<br>L | ACA<br>T | ACT<br>T | GGA<br>G | GCT<br>A | GAT<br>D | GTG<br>V | AGA<br>R |
| CAT<br>H | GAT<br>E | ATA<br>I | CCA<br>P | GTG<br>V | TTG<br>L | CCA<br>P | AAC<br>N | AGA<br>R |
| GTT<br>V | GGT<br>G | TTG<br>L | CCT<br>P | ATA<br>I | AAC<br>N | CAA<br>Q | CGG<br>R | TTT<br>F |
| ATT<br>I | TTA<br>L | GTT<br>V | GAA<br>E | CTC<br>L | TCA<br>S | AAT<br>N | CAT<br>H | GCA<br>A |
| GAG<br>E | CTT<br>L | TCT<br>S | GTT<br>V | ACA<br>T | TTA<br>L | GCC<br>A | CTG<br>L | GAT<br>D |
| GTC<br>V | ACC<br>T | AAT<br>N | GCA<br>A | TAT<br>Y | GTG<br>V | GTC<br>V | GGC<br>G | TAC<br>Y |
| CGT<br>R | GCT<br>A | GGA<br>G | AAT<br>N | AGC<br>S | GCA<br>A | TAT<br>Y | TTC<br>F | TTT<br>F |
| CAT<br>H | CCT<br>P | GAC<br>D | AAT<br>N | CAG<br>Q | GAA<br>E | GAT<br>D | GCA<br>A | GAA<br>E |
| GCA<br>A | ATC<br>I | ACT<br>T | CAT<br>H | CTT<br>L | TTC<br>F | ACT<br>T | GAT<br>D | GTT<br>V |
| CAA<br>Q | AAT<br>N | CGA<br>R | TAT<br>Y | ACA<br>T | TTC<br>F | GCC<br>A | TTT<br>F | GGT<br>G |

| | | | | | | | | |
|---|---|---|---|---|---|---|---|---|
| GGT<br>G | AAT<br>N | TAT<br>Y | GAT<br>D | AGA<br>R | CTT<br>L | GAA<br>E | CAA<br>Q | CTT<br>L |
| GCT<br>A | GGT<br>G | AAT<br>N | CTG<br>L | AGA<br>R | GAA<br>E | AAT<br>N | ATC<br>I | GAG<br>E |
| TTG<br>L | GGA<br>G | AAT<br>N | GGT<br>G | CCA<br>P | CTA<br>L | GAG<br>E | GAG<br>E | GCT<br>A |
| ATC<br>I | TCA<br>S | GCG<br>A | CTT<br>L | TAT<br>Y | TAT<br>Y | TAC<br>Y | AGT<br>S | ACT<br>T |
| GGT<br>G | GGC<br>G | ACT<br>T | CAG<br>Q | CTT<br>L | CCA<br>P | ACT<br>T | CTG<br>L | GCT<br>A |
| CGT<br>R | TCC<br>S | TTT<br>F | ATA<br>I | ATT<br>I | TGC<br>C | ATC<br>I | CAA<br>Q | ATG<br>M |
| ATT<br>I | TCA<br>S | GAA<br>E | GCA<br>A | GCA<br>A | AGA<br>R | TTC<br>F | CAA<br>Q | TAT<br>Y |
| ATT<br>I | GAG<br>E | GGA<br>G | GAA<br>E | ATG<br>M | CGC<br>R | ACG<br>T | AGA<br>R | ATT<br>I |
| AGG<br>R | TAC<br>Y | AAC<br>N | CGG<br>R | AGA<br>R | TCT<br>S | GCA<br>A | CCA<br>P | GAT<br>D |
| CCT<br>P | AGC<br>S | GTA<br>V | ATT<br>I | ACA<br>T | CTT<br>L | GAG<br>E | AAT<br>N | AGT<br>S |
| TGG<br>W | GGG<br>G | AGA<br>R | CTT<br>L | TCC<br>S | ACT<br>T | GCA<br>A | ATT<br>I | CAA<br>Q |
| GAG<br>E | TCT<br>S | AAC<br>N | CAA<br>Q | GGA<br>G | GCC<br>A | TTT<br>F | GCT<br>A | AGT<br>S |
| CCA<br>P | ATT<br>I | CAA<br>Q | CTG<br>L | CAA<br>Q | AGA<br>R | CGT<br>R | AAT<br>N | GGT<br>G |
| TCC<br>S | AAA<br>K | TTC<br>F | AGT<br>S | GTG<br>V | TAC<br>Y | GAT<br>D | GTG<br>V | AGT<br>S |
| ATA<br>I | TTA<br>L | ATC<br>I | CCT<br>P | ATC<br>I | ATA<br>I | GCT<br>A | CTC<br>L | ATG<br>M |
| GTG<br>V | TAT<br>Y | AGA<br>R | TGC<br>C | GCA<br>A | CCT<br>P | CCA<br>P | CCA<br>P | TCG<br>S |
| TCA<br>S | CAG<br>Q | TTT[2]<br>F | TCT<br>S | TTG<br>L | CTT<br>L | ATA<br>I | AGG<br>R | CCA<br>P |

| GTG | GTA | CCA | AAT | TTT | AAT | GCT[3] | GAT | GTT |
|-----|-----|-----|-----|-----|-----|-----|-----|-----|
| V | V | P | N | F | N | A | D | V |
| TGT | ATG | GAT | CCT | GAG | CCC | ATA | GTG | CGT |
| C | M | D | P | E | P | I | V | R |
| ATC | GTA | GGT | CGA | AAT | GGT | CTA | TGT | GTT |
| I | V | G | R | N | G | L | C | V |
| GAT | GTT | AGG | GAT | GGA | AGA | TTC | CAC | AAC |
| D | V | R | D | G | R | F | H | N |
| GGA | AAC | GCA | ATA | CAG | TTG | TGG | CCA | TGC |
| G | N | A | I | Q | L | W | P | C |
| AAG | TCT | AAT | ACA | GAT | GCA | AAT | CAG | CTC |
| K | S | N | T | D | A | N | Q | L |
| TGG | ACT | TTG | AAA | AGA | GAC | AAT | ACT | ATT |
| W | T | L | K | R | D | N | T | I |
| CGA | TCT | AAT | GGA | AAG | TGT | TTA | ACT | ACT |
| R | S | N | G | K | C | L | T | T |
| TAC | GGG | TAC | AGT | CCG | GGA | GTC | TAT | GTG |
| Y | G | Y | S | P | G | V | Y | V |
| ATG | ATC | TAT | GAT | TGC | AAT | ACT | GCT | GCA |
| M | I | Y | D | C | N | T | A | A |
| ACT | GAT | GCC | ACC | CGC | TGG | CAA | ATA | TGG |
| T | D | A | T | R | W | Q | I | W |
| GAT | AAT | GGA | ACC | ATC | ATA | AAT | CCC | AGA |
| D | N | G | T | I | I | N | P | R |
| TCT | AGT | CTA | GTT | TTA | GCA | GCG | ACA | TCA |
| S | S | L | V | L | A | A | T | S |
| GGG | AAC | AGT | GGT | ACC | ACA | CTT | ACG | GTG |
| G | N | S | G | T | T | L | T | V |
| CAA | ACC | AAC | ATT | TAT | GCC | GTT | AGT | CAA |
| Q | T | N | I | Y | A | V | S | Q |
| GGT | TGG | CTT | CCT | ACT | AAT | AAT | ACA | CAA |
| G | W | L | P | T | N | N | T | Q |
| CCT | TTT | GTT | ACA | ACC | ATT | GTT | GGG | CTA |
| P | F | V | T | T | I | V | G | L |

| | | | | | | | | |
|---|---|---|---|---|---|---|---|---|
| TAT Y | GGT G | CTG L | TGC C | TTG L | CAA Q | GCA A | AAT N | AGT S |
| GGA G | CAA Q | GTA V | TGG W | ATA I | GAG E | GAC D | TGT C | AGC S |
| AGT S | GAA E | AAG K | GCT A | GAA E | CAA Q | CAG Q | TGG W | GCT A |
| CTT L | TAT Y | GCA A | GAT D | GGT G | TCA S | ATA I | CGT R | CCT P |
| CAG Q | CAA Q | AAC N | CGA R | GAT D | AAT N | TGC C | CTT L | ACA T |
| AGT S | GAT D | TCT S | AAT N | ATA I | CGG R | GAA E | ACA T | GTT V |
| GTT V | AAG K | ATC I | CTC L | TCT S | TGT C | GGC G | CCT P | GCA A |
| TCC S | TCT S | GGC G | CAA Q | CGA R | TGG W | ATG M | TTC F | AAG K |
| AAT N | GAT D | GGA G | ACC T | ATT I | TTA L | AAT N | TTG L | TAT Y |
| AGT S | GGA G | TTG L | GTG V | TTA L | GAT N | GTG V | AGG R | CGA R |
| TCG S | GAT D | CCG P | AGC S | CTT L | AAA K | CAA Q | ATC I | ATT I |
| CTT L | TAC Y | CCT P | CTC L | CAT H | GGT G | GAC D | CCA P | AAC N |
| CAA Q | ATA I | TGG W | TTA L | CCA P | TTA L | TTT F | | |

Figure 2

```
           T.G A T C A C T G C A.G
              · · · · · · · · ·     · · · · · · · · ·
     T C G T A C T A G.T G.A C G T C A G C T
              ↑                    ·       ·                        ↑
           Hgi AI                 BclI PstI              Sal I
        complementary                                Complementary
          sequence                                     sequence
```

Figure 3

```
                        ala    asp    val    cys    met
     CGA    TCC    ATG    GCT    GAC    GTC    TGT    ATG
       T    AGG    TAC    CGA    CTG    CAG    ACA    TAC    CTAG
       ↑                                ↑                               ↑
     Cla I                            AatII                          Bam HI
  complementary                                                  complementary
    sequence                                                        sequence
```

Figure 4

```
                        ile    phe    pro    lys    gln    tyr    pro
     CGACG    ATG    ATC    TTT    CCG    AAA    CAG    TAT    CCG
       TGC    TAC    TAG    AAA    GGC    TTT    GTC    ATA    GGC
       ↑
  AccI/ClaI
 complementary
   sequence
```

```
       ile    ile    asn    phe    thr    thr    ala
       ATC    ATT    AAC    TTT    ACT    ACC    GCT    G
       TAG    TAA    TTG    AAA    TGA    TGG    CGA    CCACG
                                                           ↑
                                                        Ban I
                                                   complementary
                                                     sequence
```

Figure 5

```
     C G A C G A T G
       T G C T A C C T A G
```

Figure 6

7/9

0169006

Figure 7

Figure 8

Figure 9